# EUROPEAN PATENT SPECIFICATION

(11) **EP 2 822 620 B1**
(45) Date of publication and mention of the grant of the patent: **25.04.2018**
(21) Application number: 13707407.6
(22) Date of filing: 06.03.2013
(51) Int. Cl.: A61M 5/32

(54) **NEEDLE ASSEMBLY REMOVAL DEVICE AND DISPOSAL DEVICE**
NADELANORDNUNGSENTFERNUNGSVORRICHTUNG UND ENTSORGUNGSVORRICHTUNG
DISPOSITIF DE SUPPRESSION D'UN ENSEMBLE FORMANT AIGUILLE ET DISPOSITIF DE DÉPÔT

(30) Priority: 07.03.2012 EP 12158497
(43) Date of publication of application: 14.01.2015
(73) Proprietor: Sanofi-Aventis Deutschland GmbH, 65929 Frankfurt am Main (DE)
(72) Inventor: DASBACH, Uwe, 65926 Frankfurt am Main (DE)
(74) Representative: Finger, Catrin
(86) International application number: PCT/EP2013/054495
(87) International publication number: WO 2013/131952

(56) References cited:
- WO-A1-2010/113388
- WO-A2-2010/124974
- JP-A- 2009 225 825
- US-A- 5 188 598
- US-A- 5 312 346
- US-A- 5 573 113

## Description

### Technical Field

The invention relates to a device for removing a needle assembly from a medicament delivery device and a disposal device for safely discarding the removed needle assembly.

### Background of the Invention

Administering a medicament by injection is a process which presents a number of risks and challenges for patients and healthcare professionals, both mental and physical. Improper handling of medicament delivery devices, before, during and after an injection, may result in needle stick injuries. In any situation in which a patient or healthcare professional is required to manually remove the needle assembly, there is a risk of needle stick injury.

Conventional needle assemblies are attached to medicament delivery devices by a threaded engagement. Thus, after an injection, either a patient or a physician/nurse will have to remove the needle assembly by unscrewing it. As long as the needle assembly must be touched, there exists the risk of a needle stick injury.

Thus, there is a need for a device for safely removing a needle assembly from a medicament delivery device and a disposal device for discarding the removed needle assembly.

US 5 573 113 A discloses a needle removal apparatus, comprising a support member having a throughbore, a tubular sleeve reciprocally mounted in the throughbore for movement from an outermost position to an innermost position, a spring normally biasing the sleeve to the outermost position, a helical slot for constraining the sleeve to rotate during movement between the outermost position and the innermost positions, and a coupling at an inner end of the sleeve for coupling to a needle hub for unthreading the needle hub from an end of a tubular barrel during movement with the sleeve to the innermost position.

JP 2009 225825 A discloses a structure for removing a syringe needle from a syringe. The syringe needle remover has a displaceable body into which a hub of the syringe needle is inserted. The displaceable body is displaced together with a slider holder having a pair of sliders by forcing the syringe into the displaceable body. The displaceable body is displaced in the direction of bringing the pair of sliders close to each other, and the hub is held between the sliders. Then, by rotating and displacing the displaceable body together with a barrel along the interior of a housing with the hub held between the sliders, the hub is screwed and removed from a casing of the syringe. After that, the syringe needle falls into a disposal tank disposed below the syringe needle remover and stored there.

US 5 312 346 A discloses a device for removing used needles from reusable syringes. The device may be operated using only one hand and requires a single downward force applied to the syringe body by the health care worker. At the lower end of the downward stroke, the collar of the needle has been twisted off the syringe body and is automatically deposited into the receptacle for used needles. The health care worker releases the force and the apparatus automatically resets itself to receive another used needle.

WO 2010/124974 A2 discloses a needle remover for removing a needle from a holder comprising a guiding member which is suitable for receiving the needle, and a gripping means which is suitable for gripping the needle when the needle is at least partly inserted in the guiding member in an inserting direction, wherein the gripping means is configured to rotate when the gripping means is moved in the inserting direction with respect to the guiding member so that the gripped needle is unscrewed from the holder when the holder is moved in the inserting direction with respect to the guiding member.

WO 2010/113388 A1 discloses a container for collecting a needle to be discarded and a part for collecting a needle to be discarded which, in dismounting of a used syringe needle unit, allow the user to dismount the used syringe needle unit without directly touching the unit by hand and without using a complex device. A container for collecting a needle to be discarded, used to collect a syringe needle section joined through screw threads to the tip of a syringe body through a holding body. The container is provided with a container body and an opening section which is located at the upper surface of the container body and in which an insertion hole into which the holding body is inserted is formed. The opening section comprises a grip mechanism for gripping the holding body, and also comprises a ball spline shaft and a bearing which allow the grip mechanism to rotationally move as the grip mechanism moves linearly in the direction in which the insertion hole of the grip mechanism extends. The holding body which is inserted into the insertion hole is separated from the syringe body when the holding body is moved in the direction in which the insertion hole extends.

### Summary of the Invention

It is an object of the present invention to provide a device for removing a needle assembly from a medicament delivery device and a disposal device for safely discarding the removed needle assembly.

In an exemplary embodiment, a needle assembly removal device according to the present invention comprises a coupling mechanism adapted to engage a disposal device, a housing, and a carriage adapted to engage a needle assembly. Axial movement of the carriage relative to the housing causes the needle assembly to disengage a medicament delivery device.

In an exemplary embodiment according to the invention, the coupling mechanism includes threads. In an exemplary embodiment, the coupling mechanism includes at least one of a bayonet mechanism, a friction fit and a snap fit.

In an exemplary embodiment, the coupling mechanism is adapted to detachably engage the disposal device.

In an exemplary embodiment according to the invention, the housing includes a proximal opening adapted to receive the medicament delivery device and a base with a distal opening.

In an exemplary embodiment, the housing includes a track adapted to engage the carriage. The track includes one or more helical grooves. The carriage includes at least one peg adapted to engage the one or more helical grooves.

In an exemplary embodiment, the carriage is adapted to rotate in a first rotational direction when the carriage moves in a first axial direction relative to the housing and the carriage is adapted to rotate in a second rotational direction when the carriage moves in a second axial direction relative to the housing.

In an exemplary embodiment, the needle assembly removal device further comprises a spring adapted to apply a biasing force to the carriage. The spring biases the carriage in the second axial direction.

In an exemplary embodiment according to the invention, the carriage includes a retaining portion adapted to engage a needle hub of the needle assembly and prevent the needle assembly from rotating relative to the carriage. The carriage includes a slot separating the retaining portion. The retaining portion is adapted to deflect radially via the slot to accommodate the needle hub. The retaining portion includes a ramped portion adapted to engage a stem on the base. The retaining portion deflects radially when the ramped portion engages the stem.

In an exemplary embodiment, a disposal device according to the present invention comprises an interface adapted to engage the needle assembly removal device and a resilient seal covering the interface. The disposal device may further comprise a cap adapted to cover the interface and the seal.

Further scope of applicability of the present invention will become apparent from the detailed description given hereinafter. However, it should be understood that the detailed description and specific examples, while indicating preferred embodiments of the invention, are given by way of illustration only, since various changes and modifications within the scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: shows an exemplary embodiment of a needle assembly removal device and a disposal device according to the present invention before use,
- Figures 2A and 2B: show an exemplary embodiment of a needle assembly removal device according to the present invention,
- Figure 3: shows an exemplary embodiment of a needle assembly removal device and a disposal device according to the present invention during use,
- Figure 4: shows an exemplary embodiment of a needle assembly removal device according to the present invention during use,
- Figure 5: shows an exemplary embodiment of a needle assembly removal device and a disposal device according to the present invention during use,
- Figure 6: shows an exemplary embodiment of a needle assembly removal device according to the present invention during use,
- Figure 7: shows an exemplary embodiment of a needle assembly removal device according to the present invention during use,
- Figure 8: shows an exemplary embodiment of a needle assembly removal device and a disposal device according to the present invention during use,
- Figure 9: shows an exemplary embodiment of a needle assembly removal device according to the present invention during use,
- Figure 10: shows an exemplary embodiment of a needle assembly removal device and a disposal device according to the present invention after use, and
- Figure 11: shows an exemplary embodiment of a needle assembly removal device and a disposal device according to the present invention after use.

Corresponding parts are marked with the same reference symbols in all figures.

### Detailed Description

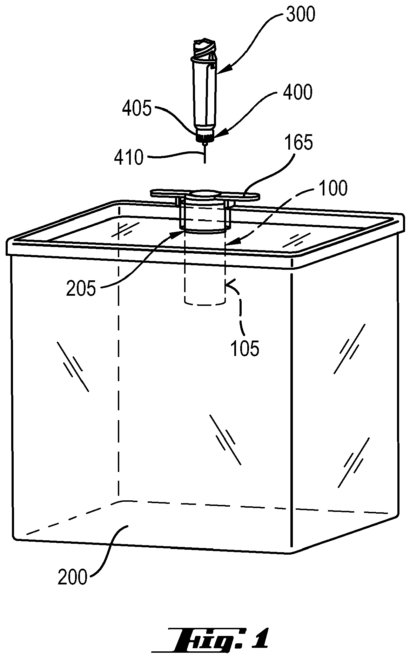

Figure 1 shows an exemplary embodiment of a needle assembly removal device 100 and a disposal device 200 according to the present invention. Figure 1 also shows a medicament delivery device 300 having a needle assembly 400 detachably coupled thereto. The medicament delivery device 300 may be a pen injector, an auto-injector, a syringe, a perfusion/infusion system, etc. The medicament delivery device 300 may contain a syringe, cartridge or ampoule filled with a medicament, or the medicament delivery device 300 may be in fluid communication with a medicament source.

In an exemplary embodiment, the needle assembly 400 includes a needle hub 405 which engages the medicament delivery device 300, and a needle 410 coupled to the needle hub 405. The needle 410 may be a double-tipped needle having a proximal tip adapted to pierce a septum on cartridge/ampoule and a distal tip adapted to pierce an injection site. In an exemplary embodiment, the needle hub 405 includes one or more threads adapted to engage a corresponding thread on the medicament delivery device 300. In other exemplary embodiments, the needle hub 405 may engage the medicament delivery device 300 via another coupling mechanism such as, for example, a bayonet mechanism, a friction fit, a snap fit, etc.

The disposal device 200 may be a sharps disposal unit which is adapted to contain used needle assemblies in a safe manner.

In an exemplary embodiment, the needle assembly removal device 100 is removably coupled to the disposal device 200. For example, the disposal device 200 may have an interface 205 (e.g., a hole) which is adapted to receive the needle assembly removal device 100 and provide access to an interior of the disposal device 200. In an exemplary embodiment, the interface 205 may have threads (or another coupling mechanism) for engaging corresponding threads on the needle assembly removal device 100. Thus, in an exemplary embodiment, the needle assembly removal device 100 may be portable. In another exemplary embodiment, the needle assembly removal device 100 may be formed integrally with the disposal device 200.

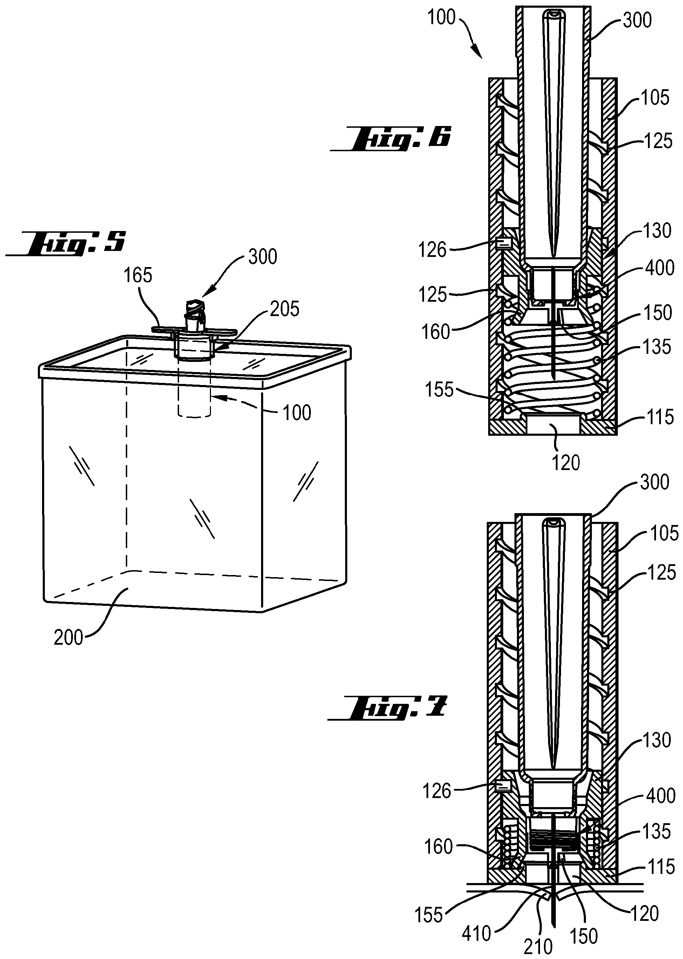

In an exemplary embodiment, the interface 205 includes a resilient seal 210 (shown in Figure 7) adapted to deflect and allow insertion of a used needle assembly into the disposal device but return to a sealed configuration after insertion. The seal 210 may prevent release of pathogens, microbes, etc. after the needle assembly removal device 100 is removed from the disposal device 200 and/or when the needle assembly removal device 100 is not in use.

While the exemplary embodiment show in Figure 1 depicts the needle assembly removal device 100 passing through the interface 205 and extending, at least partially, into the interior of the disposal device 200, those of skill in the art will understand that a distal opening of the needle assembly removal device 100 may be substantially coplanar with the seal 210. Thus, in another exemplary embodiment, the needle assembly removal device 100 may not have any portion which extends into the interior of the disposal device 200.

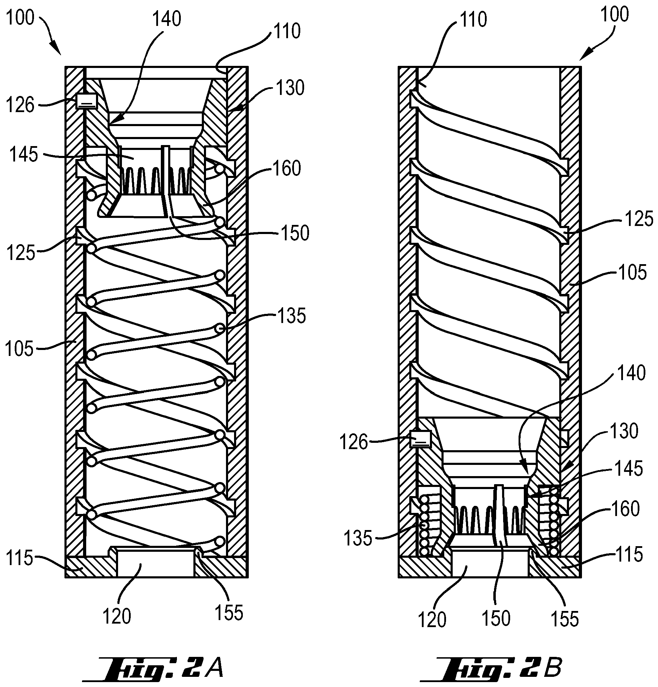

Figures 2A and 2B show an exemplary embodiment of a needle assembly removal device 100 according to the present invention. The needle assembly removal device 100 includes a housing 105, which may be cylindrical and have a proximal opening 110. A base 115 having a distal opening 120 may be formed on or coupled to a distal end of the housing 105. At least a portion of an external surface of the housing 105 may have a coupling mechanism (not shown), e.g., threads, formed thereon for engaging the corresponding coupling mechanism on the interface 205 of the disposal device 200.

A track 125 may be formed on an internal surface of the housing 105. In an exemplary embodiment, the track 125 is one or more helical grooves which extend from a proximal end of the housing 105 to the distal end.

A carriage 130 disposed in the housing 105 engages the track 125. For example, the carriage 130 may include one or more pegs 126 adapted to engage the one or more helical grooves. In the exemplary embodiment, the carriage 130 rotates in a first rotational direction when moving distally relative to the housing 105 and rotates in a second rotational direction opposite the first rotational direction when moving proximally relative to the housing 105. A spring 135 may bias the carriage 130 towards the proximal end of the housing 105.

In an exemplary embodiment, the carriage 130 is adapted to engage the needle assembly 400. For example, the carriage 130 may include a proximal guide portion 140 which is adapted to receive the needle assembly 400 and align it with a retaining portion 145. The retaining portion 145 may include a contoured surface (e.g., grooves, channels, ribs, etc.) or a frictional surface to engage the needle hub 405 and prevent rotation of the needle hub 405 relative to the carriage 130.

In an exemplary embodiment, a diameter of the retaining portion 145 may be substantially equal to or less than a diameter of the needle hub 405. The carriage 130 may include a slot 150 formed in at least a portion of the retaining portion 145. As described further below, when the needle assembly 400 is inserted into the carriage 130, the needle hub 405 may cause the retaining portion 145 to deflect via the slot 150. The retaining portion 145 may frictionally engage the needle hub 405 to prevent the needle assembly 400 from rotating relative to the carriage 130.

In an exemplary embodiment, the base 115 includes a stem 155 formed adjacent to the distal opening 120. As explained further below and shown in Figure 2B, the stem 155 engages a ramped portion 160 of the retaining portion 145 to deflect the retaining portion 145 via the slot 150, which causes the retaining portion 145 to disengage the needle assembly 400. The needle assembly 400 then passes through the interface 205 and into the interior the disposal device 200.

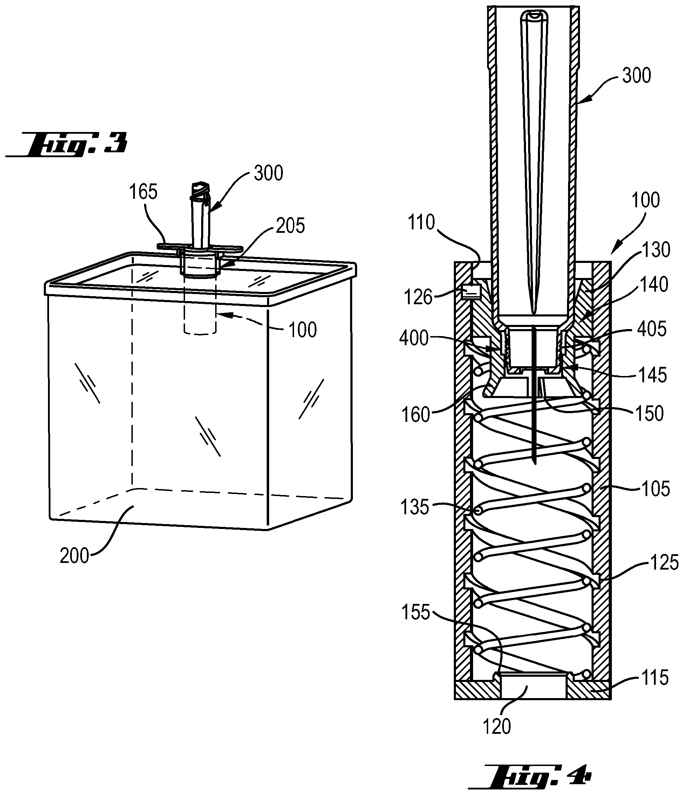

Figures 3 and 4 show an exemplary embodiment of a needle assembly removal device 100 and a disposal device 200 according to the present invention during use. In an exemplary embodiment, the needle assembly removal device 100 may have one or more handles 165 which facilitate securing the needle assembly removal device 100 to the disposal device 200. For example, the handle 165 may be used to rotate the needle assembly removal device 100 relative to the disposal device 200 to engage corresponding threaded surfaces.

As shown in Figure 4, the carriage 130 is in a first axial position (e.g., a proximal position) relative to the housing 105. When the medicament delivery device 300 is inserted into the carriage 130, the distal end of the medicament delivery device 300 may be aligned by the guide portion 140 which directs the needle assembly 400 into the retaining portion 145. When the needle hub 405 engages the retaining portion 145, the retaining portion 145 may frictionally engage the needle hub 405 and/or engage a contoured surface of the needle hub 405. For example, the retaining portion 145 may have ribs which engage grooves on the needle hub 405, or vice-versa, and/or the retaining portion 145 may deflect via the slot 150, expanding to accommodate the needle hub 405. The retaining portion 145 secures the needle hub 405 and prevents the needle assembly 400 from rotating relative to the carriage 130.

In an exemplary embodiment, a force required to compress the spring 135 is greater than a force required to deflect the retaining portion 145. Thus, the spring 135 may prevent the carriage 130 from translating relative to the housing 105 until the needle assembly 400 is secured in the carriage 130.

Figures 5, 6 and 7 show an exemplary embodiment of a needle assembly removal device 100 and a disposal device 200 according to the present invention during use. As the medicament delivery device 300 is inserted into the needle assembly removal device 100, the needle assembly 400 is disengage from the medicament delivery device 300. For example, in the exemplary embodiment shown in Figure 6, as the carriage 130 moves axially relative to the housing 105 in the distal direction, the carriage 130 rotates relative to the housing 105, because the pegs 126 on the carriage 130 follow the track 125. Because the needle assembly 400 does not rotate relative to the carriage 130, rotation of the carriage 130 causes rotation of the needle assembly 400 relative to the medicament delivery device 300, disengaging the threaded connection therebetween.

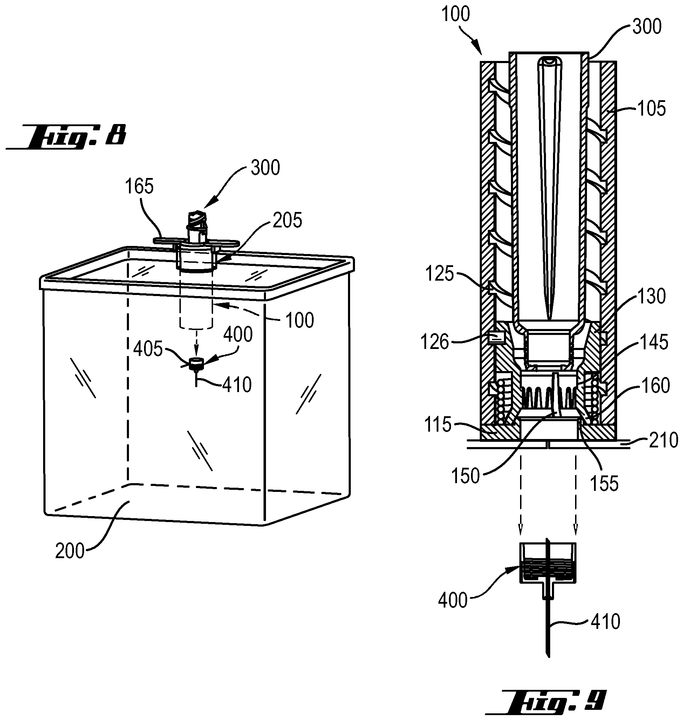

As shown in Figure 7, when the carriage 130 reaches a second axial position (e.g., a distal position) relative to the housing 105, the needle 410 deflects the seal 210. Also, the ramped portion 160 of the carriage 130 engages the stem 155, causing the retaining portion 145 to deflect via the slot 150. When the retaining portion 145 deflects, the needle assembly 400 is released from the carriage 130 and advances through the seal 210 into the interior of the disposal device 200, as shown in Figures 8 and 9. The seal 210 may return to a non-deflected position after the needle assembly 400 passes through the interface 205.

In an exemplary embodiment, a feedback (e.g., audible, tactile, visual) may be provided to indicate that the needle assembly 400 has been released from the medicament delivery device 300. For example, an exemplary audible feedback may be a sound of the ramped portion 160 snapping over the stem 155. An exemplary tactile feedback may be an increased resistance when the ramped portion 160 engages the stem 155 and/or the ramped portion 160 abutting the base 115.

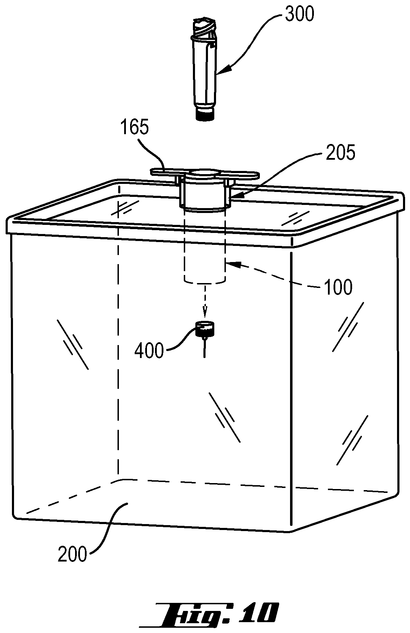

When the carriage 130 is in the second axial position, the spring 135 is compressed. Thus, when axial force being applied to the medicament delivery device 300 is released, the force in the spring 135 may be applied to the medicament delivery device 300, facilitating removal from the needle assembly removal device 100, as shown in Figure 10.

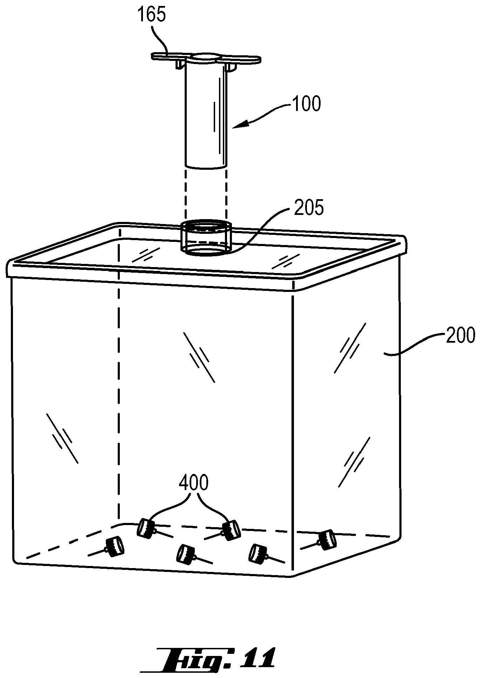

Figure 11 shows an exemplary embodiment of a needle assembly removal device 100 and a disposal device 200 according to the present invention after use. After the needle assembly 400 has been deposited in the disposal device 200 and/or after the disposal device 200 is full, the needle assembly removal device 100 may be detached from the disposal device 200. In an exemplary embodiment in which the needle assembly removal device 100 protrudes at least partially through the interface 205 into the interior of the disposal device 200, when the needle assembly removal device 100 is detached, the seal 210 may return to a non-deflected position.

In an exemplary embodiment, the disposal device 200 may include a cap (not shown) to be disposed over the interface 205 when the needle assembly removal device 100 is detached. The cap may prevent needle assemblies within the disposal device 200 from protruding through the interface 205 during, for example, transport of the disposal device 200, or if the disposal device 200 should tilt or turn-over.

Those of skill in the art will understand that modifications (additions and/or removals) of various components of the apparatuses, methods and/or systems and embodiments described herein may be made without departing from the full scope of the present invention, which encompass such modifications and any and all equivalents thereof.

## Claims

1. A needle assembly removal device (100) comprising:
a coupling mechanism including threads, the coupling mechanism adapted to engage a disposal device;
a housing (105); and
a carriage (130) adapted to engage a needle assembly (400),
wherein axial movement of the carriage (130) relative to the housing (105) causes the needle assembly (400) to disengage a medicament delivery device (300), wherein the carriage (130) is adapted to rotate in a first rotational direction when the carriage (130) moves in a first axial direction relative to the housing (105) and the carriage (13) is adapted to rotate in a second rotational direction when the carriage (130) moves in a second axial direction relative to the housing (105),
the housing (105) including a proximal opening (110) adapted to receive the medicament delivery device (300) and a base (115) with a distal opening (120), wherein the carriage (130) includes a retaining portion (145) adapted to engage a needle hub (405) of the needle assembly (400) and prevent the needle assembly (400) from rotating relative to the carriage (130),
**characterized in that** the carriage (130) includes a slot (150) separating the retaining portion (145), wherein the retaining portion (145) is adapted to deflect radially via the slot (150) to accommodate the needle hub (405), wherein the retaining portion (145) includes a ramped portion (160) adapted to engage a stem (155) on the base (115), and wherein the retaining portion (145) deflects radially when the ramped portion (160) engages the stem (155).

2. The needle assembly removal device (100) according to claim 1, wherein the coupling mechanism is adapted to detachably engage the disposal device.

3. The needle assembly removal device (100) according to any of the preceding claims, wherein the housing (105) includes a track (125) adapted to engage the carriage (130).

4. The needle assembly removal device (100) according to claim 3, wherein the track (125) includes one or more helical grooves.

5. The needle assembly removal device (100) according to claims 1 and 3, wherein the carriage (130) includes at least one peg (126) adapted to engage the one or more helical grooves.

6. The needle assembly removal device (100) according to any of the preceding claims, further comprising:
a spring (135) adapted to apply a biasing force to the carriage (130).

7. The needle assembly removal device (100) according to claim 6, wherein the spring (135) biases the carriage (130) in the second axial direction.

8. A disposal device (200) comprising:
an interface (205) adapted to engage the needle assembly removal device (100) according to any of the preceding claims; and
a resilient seal (210) covering the interface (205).

9. The disposal device (200) according to claim 8, further comprising:
a cap adapted to cover the interface (205) and the seal (210).

## Patentansprüche

1. Nadelbaugruppenentfernungsvorrichtung (100), umfassend:
einen Kopplungsmechanismus, der Gewinde aufweist, wobei der Kopplungsmechanismus zum Eingriff mit einer Entsorgungsvorrichtung ausgelegt ist;
ein Gehäuse (105); und
einen Schlitten (130), der zum Eingriff mit einer Nadelbaugruppe (400) ausgelegt ist,
wobei axiale Bewegung des Schlittens (130) relativ zu dem Gehäuse (105) bewirkt, dass sich die Nadelbaugruppe (400) von einer Medikamenten-Verabreichungsvorrichtung (300) löst, wobei der Schlitten (130) dafür ausgelegt ist, sich in einer ersten Drehrichtung zu drehen, wenn sich der Schlitten (130) in einer ersten axialen Richtung relativ zu dem Gehäuse (105) bewegt, und der Schlitten (13) dafür ausgelegt ist, sich in einer zweiten Drehrichtung zu drehen, wenn sich der Schlitten (130) in einer zweiten axialen Richtung relativ zu dem Gehäuse (105) bewegt, das Gehäuse (105) eine proximale Öffnung (110), die zum Aufnehmen der Medikamenten-Verabreichungsvorrichtung (300) ausgelegt ist, und eine Basis (115) mit einer distalen Öffnung (120) aufweist, wobei der Schlitten (130) einen Haltebereich (145) aufweist, der zum Eingriff mit einem Nadelansatz (405) der Nadelbaugruppe (400) und zum Verhindern der Drehung der Nadelbaugruppe (400) relativ zu dem Schlitten (130) ausgelegt ist,
**dadurch gekennzeichnet, dass** der Schlitten (130) einen Schlitz (150) aufweist, der den Haltebereich (145) trennt, wobei der Haltebereich (145) dafür ausgelegt ist, sich über den Schlitz (150) radial zu verbiegen, um den Nadelansatz (405) aufzunehmen, wobei der Haltebereich (145) einen abgeschrägten Teil (160) aufweist, der zum Eingriff mit einem Schaft (155) an der Basis (115) ausgelegt ist, und wobei sich der Haltebereich (145) radial verbiegt, wenn der abgeschrägte Teil (160) mit dem Schaft (155) in Eingriff steht.

2. Nadelbaugruppenentfernungsvorrichtung (100) gemäß Anspruch 1, wobei der Kopplungsmechanismus zum lösbaren Eingriff mit der Entsorgungsvorrichtung ausgelegt ist.

3. Nadelbaugruppenentfernungsvorrichtung (100) gemäß einem der vorstehenden Ansprüche, wobei das Gehäuse (105) eine Führung (125) aufweist, die zum Eingriff mit dem Schlitten (130) ausgelegt ist.

4. Nadelbaugruppenentfernungsvorrichtung (100) gemäß Anspruch 3, wobei die Führung (125) eine oder mehrere helikale Rillen aufweist.

5. Nadelbaugruppenentfernungsvorrichtung (100) gemäß Ansprüchen 1 und 3, wobei der Schlitten (130) wenigstens einen Stift (126) aufweist, der zum Eingriff mit der einen oder den mehreren helikalen Rillen ausgelegt ist.

6. Nadelbaugruppenentfernungsvorrichtung (100) gemäß einem der vorstehenden Ansprüche, ferner umfassend:
eine Feder (135), die zum Anlegen einer Vorspannungskraft an den Schlitten (130) ausgelegt ist.

7. Nadelbaugruppenentfernungsvorrichtung (100) gemäß Anspruch 6, wobei die Feder (135) den Schlitten (130) in die zweite axiale Richtung vorspannt.

8. Entsorgungsvorrichtung (200), umfassend:
eine Kopplung (205), die zum Eingriff mit der Nadelbaugruppenentfernungsvorrichtung (100) gemäß einem der vorstehenden Ansprüche ausgelegt ist; und
eine elastische Dichtung (210), die die Kopplung (205) bedeckt.

9. Entsorgungsvorrichtung (200) gemäß Anspruch 8, ferner umfassend:
eine Kappe, die zum Bedecken der Kopplung (205) und der Dichtung (210) ausgelegt ist.

## Revendications

1. Dispositif (100) de dépose d'un ensemble aiguille, comprenant :
un mécanisme d'accouplement comprenant des filetages, le mécanisme d'accouplement étant apte à entrer en prise avec un dispositif d'élimination ;
un boîtier (105) ; et
un chariot (130) apte à entrer en prise avec un ensemble aiguille (400),
dans lequel le mouvement axial du chariot (130) par rapport au boîtier (105) fait se désolidariser l'ensemble aiguille (400) du dispositif (300) d'administration de médicament,
dans lequel le chariot (130) est apte à tourner dans un premier sens de rotation quand il se déplace dans une première direction axiale par rapport au boîtier (105) et le chariot (13) est apte à tourner dans un second sens de rotation quand le chariot (130) se déplace dans une seconde direction axiale par rapport au boîtier (105), le boîtier (105) comprenant une ouverture proximale (110) apte à recevoir le dispositif (300) d'administration de médicament et une base (115) ayant une ouverture distale (120),
dans lequel le chariot (130) comprend une partie de retenue (145) apte à entrer en prise avec le moyeu (405) d'aiguille de l'ensemble aiguille (400) et empêcher l'ensemble aiguille (400) de tourner par rapport au chariot (130),
**caractérisé en ce que** le chariot (130) comprend une fente (150) divisant la partie de retenue (145),
dans lequel la partie de retenue (145) est apte à fléchir radialement à l'aide de la fente (150) pour accueillir le moyeu (405) d'aiguille,
dans lequel la partie de retenue (145) comprend une partie inclinée (160) apte à entrer en prise avec une tige (155) sur la base (115), et
dans lequel la partie de retenue (145) fléchit radialement quand la partie inclinée (160) entre en prise avec la tige (155).

2. Dispositif (100) de dépose d'un ensemble aiguille selon la revendication 1, dans lequel le mécanisme d'accouplement est apte à entrer en prise amovible avec le dispositif d'élimination.

3. Dispositif (100) de dépose d'un ensemble aiguille selon l'une quelconque des revendications précédentes, dans lequel le boîtier (105) comprend une piste (125) apte à entrer en prise avec le chariot (130).

4. Dispositif (100) de dépose d'un ensemble aiguille selon la revendication 3, dans lequel la piste (125) comprend une ou plusieurs rainures hélicoïdales.

5. Dispositif (100) de dépose d'un ensemble aiguille selon les revendications 1 et 3, dans lequel le chariot (130) comprend au moins un tenon (126) apte à entrer en prise avec la/les rainure(s) hélicoïdale(s).

6. Dispositif (100) de dépose d'un ensemble aiguille selon l'une quelconque des revendications précédentes, comprenant en outre un ressort (135) apte à appliquer une force de sollicitation au chariot (130).

7. Dispositif (100) de dépose d'un ensemble aiguille selon la revendication 6, dans lequel le ressort (135) sollicite le chariot (130) dans la seconde direction axiale.

8. Dispositif (200) d'élimination comprenant :
une interface (205) apte à entrer en prise avec un dispositif (100) de dépose d'un ensemble aiguille selon l'une quelconque des revendications précédentes ; et
un joint d'étanchéité (210) élastique couvrant l'interface (205).

9. Dispositif (200) d'élimination selon la revendication 8, comprenant en outre un capuchon apte à couvrir l'interface (205) et le joint (210).
